# FASCICULE DE BREVET EUROPEEN

(11) **EP 1 097 694 B1**
(45) Date de publication et mention de la délivrance du brevet: **03.11.2004**
(21) Numéro de dépôt: 00402739.7
(22) Date de dépôt: 05.10.2000
(51) Int. Cl.: A61K 7/00, A61K 7/48, A61K 7/06

(54) **Utilisation de cires pour stabiliser un actif hydrophile dispersé dans une phase huileuse**
Verwendung von Wachsen zur Stabilizierung eines hydrophilen Wirkstoffs, der in einer Ölphase dispergiert ist
Use of waxes to stabilize an hydrophilic active dispersed in an oil phase

(30) Priorité: 08.11.1999 FR 9913995
(43) Date de publication de la demande: 09.05.2001
(73) Titulaire: L'OREAL, 75008 Paris (FR)
(72) Inventeur: Roulier, Véronique, 75010 Paris (FR); Baldo, Francine, 92330 Sceaux (FR)
(74) Mandataire: Rasson, Catherine

(56) Documents cités:
- RO-A- 108 529
- US-A- 5 601 810
- US-A- 5 643 587

## Description

La présente invention se rapporte à une composition comprenant plus de 5 % d'eau et au moins un actif hydrophile dispersé dans une phase huileuse comprenant au moins 5 % en poids d'une ou plusieurs cires par rapport au poids total de la composition, à ses applications, plus particulièrement dans le domaine cosmétique, pour la libération de cet actif, notamment en vue de traiter et/ou nettoyer et/ou protéger la peau et/ou les muqueuses et/ou les fibres kératiniques.

La composition de l'invention peut être appliquée, par voie topique, sur le visage, y compris autour des yeux, sur le corps ainsi que sur le cuir chevelu des êtres humains.

Il est connu d'introduire dans les compositions cosmétiques et/ou dermatologiques des actifs en vue d'apporter des traitements spécifiques à la peau et/ou aux cheveux, par exemple pour nettoyer la peau, pour lutter contre le dessèchement, les signes du vieillissement ou la pigmentation de la peau, pour traiter l'acné ou certains désordres de la peau (eczéma, psoriasis), pour combattre les surcharges pondérales, pour favoriser la restructuration de la peau ou son renouvellement cellulaire, pour traiter la séborrhée des cheveux.

Par exemple, on cherche depuis longtemps à formuler l'acide ascorbique (ou vitamine C) dans les domaines cosmétique et dermatologique, sous différentes formes galéniques, du fait de ses nombreuses propriétés bénéfiques. En particulier, l'acide ascorbique stimule la synthèse du tissu conjonctif et notamment du collagène, renforce les défenses du tissu cutané contre les agressions extérieures telles que les rayonnements ultraviolets et la pollution, compense la déficience en vitamine E de la peau, dépigmente la peau et possède une fonction anti-radicaux libres. Du fait de ses propriétés, l'acide ascorbique est efficace pour nettoyer la peau et également pour lutter contre les signes du vieillissement de la peau, par exemple pour améliorer l'éclat du teint et estomper les ridules et/ou rides de la peau. L'acide ascorbique est également utilisé dans des compositions destinées au massage comme décrit dans le document RO-108529.

Malheureusement, ces actifs sont souvent instables en milieu oxydant et donc très sensibles à certains paramètres de l'environnement comme par exemple la lumière, l'oxygène et l'eau. Il s'ensuit donc une dégradation rapide de ces actifs lorsqu'ils sont en contact avec notamment un de ces paramètres, ce qui va à l'encontre de l'efficacité recherchée.

Dans l'état de la technique ce problème a été diversement traité. Ainsi, pour diminuer ou retarder la dégradation de l'acide ascorbique en solution, le document US-A-5,140,043 préconise de le stabiliser en l'introduisant dans des solutions hydroalcooliques, formées d'au moins 80 % d'eau et ayant un pH inférieur à 3,5. En raison de la forte acidité de ces solutions, leur utilisation dans le domaine cosmétique est difficilement envisageable. En effet, une application répétée de ces solutions peut perturber l'équilibre de la peau et en particulier irriter, voire brûler la peau.

D'autres modes de stabilisation de l'acide ascorbique ont été envisagés notamment par enrobage (technique décrite dans le document FR-A-1600826) ou par granulation de l'acide ascorbique (technique illustrée dans le document JP-A-53-127819, pour l'agro-alimentaire). Mais ces techniques sont d'une part coûteuses et peuvent d'autre part altérer l'acide ascorbique, par exemple lors d'un chauffage, et/ou conduire à des compositions peu cosmétiques, comme c'est le cas des granulés.

En outre, le document US-A-5,853,741 décrit la stabilisation de l'acide ascorbique par des silicones élastomères non-émulsifiants connus sous les références commerciales « Gransil » de la société Grand Industrie. L'inconvénient de ces produits est d'apporter un effet huileux, gras, sans effet frais, ce qui ne permet pas ou difficilement leur utilisation en milieu chaud et humide et/ou par les utilisateurs à peaux grasses. De plus ces polymères sont totalement hydrofuges et difficilement incorporables en phase aqueuse. Du fait de leur forte incompatibilité avec l'eau et en particulier avec la sueur, cette dernière n'est pas absorbée par ces polymères et a même tendance à « perler » à la surface de la peau, lorsque celle-ci transpire.

Il subsiste donc le besoin d'une composition utilisable notamment dans le domaine cosmétique, dans laquelle un actif hydrophile est stable, qui est confortable lors de l'application et qui ne provoque aucune irritation de la peau après application.

La demanderesse a maintenant trouvé une composition surmontant les inconvénients de l'art antérieur.

La présente invention a donc pour objet l'utilisation d'au moins 5 % en poids d'une ou plusieurs cires pour stabiliser un actif hydrophile choisi parmi la vitamine C et ses dérivés glycosylés et phosphatés dispersé dans la phase huileuse d'une composition comprenant plus de 5 % en poids d'eau, par rapport au poids total de la composition, et une ou plusieurs charges.

La composition de l'invention, bien que contenant un fort taux de cire, se présente sous forme d'une crème, c'est-à-dire d'un produit souple par opposition aux produits solides tels que les sticks. On entend ici par produit souple, un produit ayant une viscosité allant d'environ 1 à 25 Pa.s, ladite viscosité étant mesurée à température ambiante (environ 20-25°C) avec un Rhéomat 180.

La composition de l'invention présente l'avantage d'être fraîche à l'application.

La composition de l'invention contient, dans la phase huileuse, au moins 5 % en poids d'une ou plusieurs cires, par rapport au poids total de la composition. La phase huileuse se présente, avant l'éventuel mélange avec une phase aqueuse, sous forme d'une pâte souple. Cette pâte souple est obtenue notamment par chauffage de la cire ou les cires au delà de, ou de préférence jusqu'à, leurs points de fusion et d'éventuels d'autres constituants de la phase huileuse, suivi d'un refroidissement au cours duquel un malaxage est effectué. Ce malaxage est réalisé soit par un broyeur à cylindres, soit par un mélangeur-extrudeur à vis. Cette pâte souple est notamment décrite dans les brevets EP 0667146, EP 0706790, EP 0745376 et EP 0755668. On entend par "pâte souple" une pâte dont on peut mesurer la viscosité, par opposition à la structure solide d'un bâton ou stick, dont on ne peut pas mesurer la viscosité. La viscosité dynamique de la pâte souple à 25°C est généralement comprise entre 3 et 35 Pa.s, mesurée avec un viscosimètre rotatif CONTRAVES TV équipé d'un mobile "MS-r4" à la fréquence de 60 Hz.

Comme cires utilisables dans la composition de l'invention, on peut citer par exemple les cires minérales telles que les cires microcristallines, la paraffine, le pétrolatum, la vaseline, l'ozokérite, la cire de montan ; les cires animales telles que la cire d'abeilles, la lanoline et ses dérivés ; les cires végétales telles que les cires de Candellila, d'Ouricurry, de Carnauba, du Japon, le beurre de cacao, les cires de fibres de lièges ou de canne à sucre ; les huiles hydrogénées concrètes à 25°C ; les esters gras et les glycérides concrets à 25°C ; les cires synthétiques telles que les cires de polyéthylène et les cires obtenues par synthèse de Fischer-Tropsch ; les cires de silicone, et leurs mélanges.

Selon un mode préféré de réalisation de l'invention, on utilise au moins une cire ayant une température de fusion commençante supérieure ou égale à 50°C, et mieux au moins une cire dont la température de fusion commençante est supérieure à 65°C, telles que la cire de Carnauba, certaines cires de polyéthylène et certaines cires microcristallines telles que celle vendue par la société Tisco sous le nom "Tisco Wax 88" ou celle vendue par la société RMC sous le nom de "Feruwax 30540".

Par "température de fusion commençante", on entend dans la présente description la température à laquelle une cire commence à fondre. On peut déterminer cette température par ATD (analyse thermique différentielle) qui permet l'obtention du thermogramme (ou courbe de fusion) de la cire considérée. La température de fusion commençante correspond à la température à laquelle on peut observer un changement de pente notable dans le thermogramme. Le point de fusion, quant à lui, représente le point minimum dudit thermogramme.

La quantité de cire(s) dans la composition de l'invention est d'au moins 5 % et va de préférence de 5 à 40 % et mieux de 5 à 20 % en poids par rapport au poids total de la composition.

La phase huileuse de la composition de l'invention comprend généralement, outre la ou les cires, un ou plusieurs corps gras choisis parmi les huiles d'origine animale, les huiles d'origine végétale, les huiles minérales, les huiles synthétiques, les huiles fluorées, les huiles de silicone et notamment les huiles de silicone volatiles, les gommes de silicone, les résines de silicone, les alcools gras, les acides gras et les élastomères de silicone tels que les produits commercialisés sous la dénomination "KSG" par la société Shin-Etsu, sous la dénomination "Trefil" par la société Dow Corning ou sous la dénomination "Gransil" par la société General Electric.

La quantité de la phase huileuse, comprenant au moins 5 % en poids d'une ou plusieurs cires et l'actif hydrosoluble dispersé, va généralement de 10 à 95 % en poids par rapport au poids total de la composition selon l'invention. Lorsque la composition est une émulsion, la quantité de cette phase huileuse est comprise de préférence entre 10 et 90 %, avantageusement de 20 à 60 %, en poids par rapport au poids total de la composition. Cette phase huileuse est utilisée en une quantité telle ou bien contient une quantité de cires telle que la quantité de cires dans la composition finale est égale ou supérieure à 5 %.

La composition de l'invention contient une ou plusieurs charges (constituants pulvérulents) qui peuvent être choisies par exemple dans le groupe formé par le talc ; les micas d'origine naturelle ou synthétique ; le kaolin ; les oxydes de zinc ou de titane ; le carbonate de calcium ; le carbonate et l'hydrocarbonate de magnésium ; la silice, en particulier la silice sphérique, la poudre de silice commercialisée sous la dénomination "Cab-O-Sil TS 530" par la société Cabot, et les microbilles de silice telles que celles commercialisées sous la dénomination SB150 par la société Myoshi ; le dioxyde de titane ; les billes de verre et de céramique commercialisées par la société 3M sous la dénomination commerciale "Macrolite" ; les savons métalliques dérivés d'acide organique carboxylique ayant de 8 à 22 atomes de carbone, de préférence de 12 à 18 atomes de carbone, par exemple le stéarate de zinc, de magnésium ou de lithium, le laurate de zinc, le myristate de magnésium ; les poudres de polymères synthétiques non expansées, telles que les poudres de polyéthylène, de polystyrène, de polyesters, de polyamides (par exemple le nylon ou la poly-β-alanine), de copolymères d'acrylates (par exemple les microsphères microporeuses vendues par la société Dow Corning sous la dénomination commerciale "Polytrap"), d'acides polyméthacryliques, de polystyrène, de téflon comme le "Fluon" ; les poudres expansées telles que les microsphères creuses en matériau thermoplastique préparées par des procédés connus, comme ceux décrits dans US-A-3 615 972 et EP-A-0 56219 et notamment les microsphères commercialisées sous la dénomination commerciale "Expancel" par la société Kemanord Plast ou sous la dénomination commerciale "Micropearl F 80 ED" par la société Matsumoto ; les poudres de matériaux organiques naturels tels que les amidons de maïs, de blé ou de riz, réticulés ou non, telles que les poudres d'amidon réticulé par l'anhydride octénylsuccinique, commercialisées sous la dénomination "Dry-Flo" par la société National Starch ; les microbilles de résine de silicone telles que celles commercialisées sous la dénomination "Tospearl" par la société Toshiba Silicone, et leurs mélanges.

Les charges représentent jusqu'à 20 % en poids par rapport au poids total de la composition, et de préférence de 1 à 12 % en poids par rapport au poids total de la composition.

L'utilisation de l'invention permet de stabiliser un actif hydrophile, instable en milieu oxydant, et sensible à l'eau, à l'air ou aux métaux, choisi parmi l'acide ascorbique (vitamine C) et ses dérivés glycosylés et phosphatés, et leurs mélanges.

Il peut s'agir également de tous les composés hydrophiles naturels ou synthétiques pouvant contenir les actifs indiqués ci-dessus, en particulier les extraits végétaux, et plus spécialement les extraits de fruits.

L'utilisation de l'invention est particulièrement intéressante pour stabiliser la vitamine C. Ainsi, dans la composition selon l'invention, les pertes en acide ascorbique après 6 mois à température ambiante (20 à 25°C) sont inférieures à 20 % et de préférence inférieure à 10 %, alors que, dans une composition classique, la perte atteint 100 % dans les mêmes conditions de temps et de température.

La quantité d'actif hydrophile dans la composition selon l'invention dépend du type d'actif utilisé et du but recherché. De manière générale, le ou les actifs peuvent être utilisés dans la composition selon l'invention en une quantité allant de 0,01 à 20 % en poids, de préférence de 0,04 à 15 %, et mieux de 0,1 à 10 % en poids par rapport au poids total de la composition.

L'actif hydrophile est introduit dans la phase huileuse de la composition. Ainsi, cet actif hydrophile se trouve dispersé dans la phase huileuse. Il est incorporé dans cette phase huileuse par mélange de cette phase avec l'actif qui peut être sous forme pulvérulente ou liquide.

Ainsi, l'acide ascorbique pulvérulent utilisable dans invention peut être celui vendu par Hoffmann-La Roche; il se présente sous forme de l'acide L-ascorbique avec une pureté de 99% à 100%. Il serait bien entendu possible d'utiliser de l'acide D-ascorbique pulvérulent pur à 99% au moins.

La composition comprend également plus de 5 % en poids d'eau par rapport à son poids total. De préférence, elle comprend au moins 7% en poids d'eau, avantageusement au moins 10 % d'eau.

La composition selon l'invention se présente sous forme d'une crème et elle peut avoir différentes formes galéniques. Elle peut en particulier se trouver sous la forme d'une dispersion ou de préférence sous la forme d'une émulsion, telle que notamment une émulsion huile-dans-eau ou eau-dans-huile. On entend ici par "émulsion" aussi bien des dispersions sans émulsionnants que des dispersions comportant des émulsionnants ou encore des dispersions stabilisées par des particules solides ou par des sphérules lipidiques de type ionique ou non ionique.

Lorsque la composition est une émulsion, la quantité de la phase aqueuse est comprise de préférence entre 10 et 90 %, avantageusement de 40 à 80 %, en poids par rapport au poids total de la composition.

Par ailleurs, la composition de l'invention peut être plus ou moins fluide.

Lorsque la composition est une émulsion huile-dans-eau, la composition comprend avantageusement un système émulsionnant. Ce système émulsionnant est avantageusement choisi parmi (1) un mélange d'au moins deux émulsionnants non ioniques, ledit mélange étant liquide à la température ambiante et ayant un HLB allant de 6 à 13 et (2) au moins un émulsionnant anionique liquide à température ambiante.

Par température ambiante, on entend selon l'invention une température allant de 15°C à 25°C.

Le mélange d'émulsionnants non ioniques, liquide à la température ambiante et ayant un HLB allant de 6 à 13, utilisable dans la composition de l'invention, peut comprendre notamment (1) au moins un émulsionnant non ionique ayant un HLB égal ou supérieur à 13 et (2) au moins un émulsionnant non ionique ayant un HLB égal ou inférieur à 5. En outre, il peut comprendre éventuellement au moins un co-émulsionnant qui peut être notamment nécessaire si les émulsionnants (1) et (2) utilisés sont tous deux solides, le co-émulsionnant étant alors liquide et permettant d'obtenir un mélange liquide.

De manière connue, le HLB d'un mélange d'émulsionnants correspond à la moyenne des HLB de chacun des émulsionnants constituant le mélange, compte tenu de la proportion pondérale de ces émulsionnants.

Comme émulsionnants non ioniques de HLB égal ou inférieur à 5, on peut citer par exemple les esters de polyols et d'acide gras ayant une chaîne alkyle comportant de 12 à 22 atomes de carbone, tels que les esters d'acide gras et de glycérine, de glucose ou de sorbitol ; les dérivés oxyéthylénés des esters de polyols et d'acide gras ayant une chaîne alkyle comportant de 12 à 22 atomes de carbone, comportant de 1 à 50 groupes oxyéthylénés, tels que le complexe de triisostéarine (triester de glycérine et d'acide isostéarique) et de PEG-6 ; les éthers de polyéthylène glycol et d'alcool gras ayant une chaîne alkyle comportant de 12 à 22 atomes de carbone, comportant de 1 à 50 groupes oxyéthylénés, tels que les éthers d'oléyle et en particulier l'oleth-25 (25 groupes oxyéthylénés), et leurs mélanges.

Comme émulsionnants non ioniques de HLB égal ou inférieur à 5, on peut utiliser avantageusement ceux qui sont liquides à température ambiante, tels que les esters gras et éthers gras de polyol, à chaîne ramifiée ou insaturée comportant de 12 à 22 atomes de carbone, et en particulier le mono-isostéarate de sorbitan comme le produit vendu sous la dénomination "Arlacel 987" par la société ICI, le mono/di-oléate de sorbitan comme le produit vendu sous la dénomination "Arlacel 83" par la société ICI, le complexe de triisostéarine et de PEG-6 comme le produit vendu sous la dénomination "Labrafil isostearique" par la société Gattefossé, le penta-isostéarate de décaglycéryle comme le produit vendu sous la dénomination "Nikkol Decaglyn 5-IS" par la société Nikko Chemical, le dioléate de méthylglucose comme le produit vendu sous la dénomination "Isolan DO" par la société Goldschmidt.

Comme émulsionnants non ioniques de HLB égal ou supérieur à 13, on peut citer par exemple les esters de polyéthylène glycol et d'acide gras ayant une chaîne alkyle comportant de 12 à 22 atomes de carbone, comportant de 5 à 100 et de préférence de 20 à 60 groupes oxyéthylénés, tels que le stéarate de PEG-40 ; les éthers de polyéthylène glycol et d'alcool gras ayant une chaîne alkyle comportant de 12 à 22 atomes de carbone, comportant de 5 à 100 et de préférence de 10 à 30 groupes oxyéthylénés, tels que le ceteareth-25 ou le ceteth-25 ; les esters de sorbitan et d'acide gras ayant une chaîne alkyle comportant de 12 à 22 atomes de carbone, comportant de 0 à 100 et de préférence de 4 à 25 groupes oxyéthylénés, tels que le Polysorbate 20, le Polysorbate 40 et le Polysorbate 60 ; les esters de sucre et d'acide gras ayant une chaîne alkyle comportant de 12 à 22 atomes de carbone, tels que le stéarate de sucrose ; les dérivés de polyéthylène glycol et d'esters de glycérine et d'acide gras ayant une chaîne alkyle comportant de 12 à 22 atomes de carbone, tels que le PEG-8 caprylic/capric glycerides ; les éthers de polyéthylène glycol et des esters de méthyl glucose et d'acide gras ayant une chaîne alkyle comportant de 12 à 22 atomes de carbone, tels que le PEG-20 methyl glucose sesquistéarate ; et leurs mélanges.

Comme émulsionnants non ioniques de HLB égal ou supérieur à 13, on peut utiliser avantageusement ceux qui sont liquides à température ambiante, tels que le Polysorbate 20 comme le produit vendu sous la dénomination "Tween 20" par la société ICI, le Polysorbate 40 comme le produit vendu sous la dénomination "Tween 40" par la société ICI, le PEG-8 caprylic/capric glycerides comme le produit vendu sous la dénomination "Labrasol" par la société Gattefossé, le PEG-20 methyl glucose sesquistéarate comme le produit vendu sous la dénomination "Glucamate SSE 20" par la société Amerchol.

Comme co-émulsionnant, on peut citer par exemple les alcools gras comportant une chaîne ramifiée ou insaturée ayant de 8 à 22 atomes de carbone, tels que l'alcool isostéarique ; les acides gras comportant une chaîne ramifiée ou insaturée ayant de 8 à 22 atomes de carbone, tels que l'acide ricinoléique ; les esters de polyol et d'acide gras ramifié comportant de 8 à 22 atomes de carbone, tels que les esters gras ramifiés de glycéryle ou de propylène glycol comme l'isostéarate de glycéryle ou l'isostéarate de propylène glycol, et leurs mélanges.

Comme mélange d'émulsionnants non ioniques, liquide à température ambiante et ayant un HLB de 6 à 13, utilisable dans la composition selon l'invention, on peut citer par exemple le mélange de stéarate de glycéryle, stéarate de propylène glycol, isostéarate de glycéryle, isostéarate de propylène glycol, oleth-25 et ceteth-25, commercialisé par la société Gattefosse sous la dénomination "Hydrolactol 70" de HLB 10.

La quantité de mélange d'émulsionnants non ioniques, liquide à la température ambiante et ayant un HLB allant de 6 à 13, et éventuellement de co-émulsionnant, dans la composition selon l'invention va généralement de 0,1 à 30 % en poids en matière active et de préférence de 1 à 25 % en poids en matière active par rapport au poids total de la composition. Dans cette quantité d'émulsionnants, la proportion de chaque émulsionnant peut être aisément déterminée par l'homme du métier en vue d'obtenir un mélange adapté aux critères définis ci-dessus. De manière préférée, la quantité d'émulsionnants non ioniques sans tenir compte du co-émulsionnant va de 0,5 à 10 % en poids et de préférence de 0,5 à 5 % en poids par rapport au poids total de la composition.

L'émulsionnant anionique liquide à température ambiante utilisable dans la composition selon l'invention peut être choisi notamment dans le groupe des tensioactifs anioniques à groupement phosphate, liquides à la température ambiante, tels que les mono-, di- et/ou tri-esters d'acide phosphorique et d'alcool gras en C₁₂ à C₂₂, c'est-à-dire ayant une chaîne alkyle comportant de 12 à 22 atomes de carbone, et les mono, di- et/ou tri-esters d'acide phosphorique et d'alcool gras éthoxylé, ayant une chaîne grasse comportant de 12 à 22 atomes de carbone et un nombre de motifs éthoxylés (=oxyéthylénés) allant de 1 à 100 et de préférence de 4 à 25.

On peut citer notamment comme émulsionnant anionique liquide à la température ambiante, le phosphate d'oléyle (mélange de mono- et de diester d'acide phosphorique et d'alcool oléique), le phosphate de trioléyle, le phosphate de dilaureth-4 (mélange de diesters d'acide phosphorique et d'éther de polyéthylène glycol et d'alcool laurique, ayant 4 groupes oxyéthylénés), le phosphate de trioleth-8 (mélange de triesters d'acide phosphorique et d'éther de polyéthylène glycol et d'alcool oléique, ayant 8 groupes oxyéthylénés), le phosphate de triceteareth-4 (mélange de triesters d'acide phosphorique et d'éther de polyéthylène glycol et d'alcools cétylique et stéarylique, ayant 4 groupes oxyéthylénés), le mono-ester d'acide phosphorique et d'acides stéarique et isostéarique (nom CTFA : octyldecyl phosphate) vendu sous la dénomination Hostaphat CG 120 par la société Clariant, et leurs mélanges.

Selon un mode préféré de réalisation de l'invention, on utilise, comme émulsionnant anionique liquide à température ambiante, l'octyldecyl phosphate.

La quantité d'émulsionnant anionique liquide à la température ambiante dans la composition selon l'invention va généralement de 0,1 à 10 % en poids en matière active et de préférence de 1 à 5 % en poids en matière active par rapport au poids total de la composition.

On peut éventuellement ajouter à l'émulsionnant anionique liquide à la température ambiante, un co-émulsionnant du moment que le mélange de l'émulsionnant et du co-émulsionnant est liquide à température ambiante.

Ainsi, comme co-émulsionnant, on peut citer par exemple les alcools gras comportant une chaîne ramifiée ou insaturée ayant de 8 à 22 atomes de carbone, tels que l'alcool isostéarique, les acides gras comportant une chaîne ramifiée ou insaturée ayant de 8 à 22 atomes de carbone, tels que l'acide ricinoléique, et leurs mélanges.

Dans cette variante, quand la composition contient un co-émulsionnant, la quantité de co-émulsionnant va généralement de 0,1 à 10 % en poids en matière active et de préférence de 1 à 5 % en poids en matière active par rapport au poids total de la composition.

Lorsque la composition est une émulsion eau-dans-huile, la composition contient avantageusement au moins 25 % en poids de phase aqueuse par rapport au poids total de la composition et un système émulsionnant comprenant au moins un émulsionnant siliconé. Dans cette variante, la phase aqueuse de la composition de l'invention représente au moins 25 % en poids par rapport au poids total de la composition et de préférence de 40 à 70 % en poids par rapport au poids total de la composition.

L'émulsionnant siliconé utilisable dans la composition de l'invention est alors généralement choisi dans le groupe comprenant les polydiméthylsiloxanes oxyéthylénés et/ou oxypropylénés, les alkyl-C₁₀-C₂₂-polydiméthylsiloxanes oxyéthylénés et/ou oxypropylénés ("alkyl-C₁₀-C₂₂" signifiant que la chaîne alkyle comporte de 10 à 22 atomes de carbone), les polydiméthylsiloxanes oxyéthylénés et/ou oxypropylénés à groupements glucosides, et leurs mélanges. Ces polydiméthylsiloxanes peuvent être éventuellement réticulés.

L'émulsionnant siliconé peut être introduit tel quel ou en mélange avec une huile de silicone volatile ou non volatile, telle qu'une cyclométhicone (cyclohexasiloxane, cyclopentasiloxane, cyclotétrasiloxane).

On peut citer comme émulsionnant siliconé utilisable dans la composition de l'invention, les mélanges de dimethicone copolyol et de cyclomethicone vendus sous les dénominations "Q2-3225C" et "DC2-5225C" par la société Dow Corning, le produit vendu sous la dénomination "SF-1288" par la Société General Electric, le mélange de Polyglyceryl-4 isostearate/Cetyl dimethicone copolyol/Hexyl laurate vendu sous la dénomination "Abil WE 09" par la société Goldschmidt, le Cetyl dimethicone copolyol vendu sous la dénomination "Abil EM 90" par la société Goldschmidt, le Laurylmethicone copolyol vendu sous la dénomination "Q2-5200" par la société Dow Corning.

La quantité d'émulsionnant siliconé dans la composition selon l'invention va généralement de 0,1 à 10 % en poids en matière active et de préférence de 2 à 5 % en poids en matière active par rapport au poids total de la composition.

La composition selon l'invention présente en outre l'avantage d'être stable, c'est-à-dire dans le cas d'une émulsion, de ne pas se déphaser.

De façon avantageuse, cette composition peut être utilisée comme composition cosmétique et/ou dermatologique.

La composition de l'invention peut se présenter sous forme d'un produit à appliquer sur les lèvres, les yeux, la peau et/ou les phanères d'êtres humains. Elle contient donc un milieu physiologiquement acceptable, c'est-à-dire un milieu compatible avec toutes les matières kératiniques telles que la peau aussi bien du corps humain que du visage, les ongles, les cheveux, les cils et les sourcils.

Comme indiqué précédemment, les compositions selon l'invention peuvent être utilisées dans différentes applications topiques, notamment cosmétiques et/ou dermatologiques. La composition selon l'invention peut constituer notamment des compositions de nettoyage, de protection, de traitement ou de soin de la peau et/ou des cheveux, en particulier pour le visage, pour le cou, pour les mains, pour les cheveux, pour le cuir chevelu ou pour le corps, ainsi que pour les cils.

La composition selon l'invention peut être utilisée pour la préparation d'une composition destinée à traiter et/ou nettoyer et/ou protéger la peau et/ou les muqueuses et/ou les fibres kératiniques, c'est-à-dire les cheveux et/ou les cils.

La composition selon l'invention peut être utilisée dans un procédé cosmétique pour nettoyer et/ou traiter et/ou protéger la peau, les muqueuses et/ou les fibres kératiniques, caractérisé en ce qu'il comprend l'application sur la peau et/ou les muqueuses et/ou les fibres kératiniques, d'une composition telle que définie ci-dessus.

La composition selon l'invention peut constituer notamment des crèmes de protection, de traitement ou de soin pour le visage, pour les mains, pour les pieds, des laits corporels de protection ou de soin, des lotions, gels ou mousses pour le soin de la peau, des muqueuses, des cheveux et du cuir chevelu.

De façon connue, la composition de l'invention peut contenir également des adjuvants habituels dans les domaines cosmétique et dermatologique, tels que les tensioactifs, notamment les tensioactifs moussants, les actifs hydrophiles ou lipophiles, les conservateurs, les antioxydants, les séquestrants, les solvants, les parfums, les filtres, les absorbeurs d'odeur et les matières colorantes. Les quantités de ces différents adjuvants sont celles classiquement utilisées dans les domaines considérés, et par exemple de 0,01 % à 15 % du poids total de la composition. Elle peut contenir également des vésicules lipidiques formées de lipides ioniques ou non ioniques.

Ces adjuvants, selon leur nature, peuvent être introduits dans la phase grasse ou dans la phase aqueuse.

Comme actifs hydrophiles, on peut utiliser par exemple, les polyols, les protéines ou les hydrolysats de protéine, les acides aminés, les hydroxy-acides, l'allantoïne, les sucres et les dérivés de sucre, l'amidon.

Comme actifs lipophiles, on peut utiliser par exemple le tocophérol (vitamine E) et ses dérivés, les acides gras essentiels, les céramides, les huiles essentielles, le rétinol et ses dérivés.

Bien entendu, l'homme du métier veillera à choisir ce ou ces éventuels adjuvants, et/ou leur quantité, de manière telle que les propriétés avantageuses de la composition selon l'invention ne soient pas, ou substantiellement pas, altérées par l'adjonction envisagée.

La composition selon l'invention peut être préparée de manière avantageuse en utilisant, pour au moins une étape du procédé, un appareil de malaxage tel qu'un broyeur à cylindres comportant deux cylindres tournant en sens inverse entre lesquels passe la pâte ou un mélangeur-extrudeur à vis. On utilise de préférence un mélangeur-extrudeur à vis.

Un autre objet de l'invention est donc un procédé de préparation d'une composition selon l'invention, caractérisé en ce que l'on effectue au moins une étape du procédé à l'aide d' un broyeur à cylindres ou d'un mélangeur-extrudeur à vis, de préférence d'un mélangeur-extrudeur à vis.

Selon un premier mode de réalisation de l'invention, le procédé de préparation comprend les étapes suivantes :
- (1) préparation de la phase huileuse sous forme d'une pâte souple obtenue par :
   (a) chauffage de la cire ou les cires au delà de, ou de préférence jusqu'à, leurs points de fusion et d'éventuels d'autres constituants, tels que les corps gras, de la phase huileuse,
   (b) un refroidissement jusqu'à température ambiante (15°C à 25°C) au cours duquel un malaxage est effectué, l'actif hydrophile et éventuellement d'autres constituants (notamment les charges) de la phase huileuse, étant introduits lors de cette étape (b), et en malaxant le mélange obtenu tout en le refroidissant jusqu'à température ambiante tandis qu'il est amené à la sortie du mélangeur-extrudeur,
- (2) éventuellement incorporation d'un système émulsionnant dans la pâte souple obtenue en (1), et
- (3) éventuellement incorporation, sous agitation, du mélange obtenu en (2) dans la phase aqueuse ou inversement (incorporation de la phase aqueuse dans le mélange obtenu en (2)).

Le malaxage est donc avantageusement réalisé soit par un broyeur à cylindres, soit par un mélangeur-extrudeur à vis.

De préférence, l'actif hydrosoluble est mélangé avec les charges préalablement à leur introduction à l'étape 1(b).

Dans ce mode de réalisation, les étapes (2) et (3) sont uniquement réalisées lorsque l'on désire obtenir une émulsion simple de type huile-dans-eau ou inversement eau-dans-huile ; celles-ci sont mises en oeuvre dans un appareil de mélange habituellement utilisé par l'homme du métier, tel qu'un rotor stator.

En outre, dans le procédé décrit ci-dessus, les quantités utilisées sont telles que la composition finale comprenne au moins 5 % de cire en poids.

Comme indiqué plus haut, le mélange des phase huileuse et aqueuse se faisant à température ambiante (ou à froid), l'incorporation de composés thermosensibles ne pose pas de problème.

Selon un mode particulier de réalisation de l'invention, les étapes (2) et (3) ci-dessus sont également réalisées dans le mélangeur-extrudeur à vis ou le broyeur à cylindres utilisé pour l'étape (1). Le système émulsionnant et la phase aqueuse sont alors introduits dans une partie (ou élément) du mélangeur-extrudeur où la température est proche de la température ambiante.

L'utilisation d'un mélangeur-extrudeur permet d'obtenir une pâte de phase huileuse de qualité très constante de façon reproductible. De plus, il est possible, en adaptant la filière de sortie du mélangeur-extrudeur, de conditionner la composition en ligne à la sortie dudit mélangeur-extrudeur.

Les différents étapes du procédé peuvent être réalisées dans un ou plusieurs extrudeurs disposés à la suite les uns des autres, et de préférence dans un extrudeur bivis unique.

Les différentes conditions dans lesquelles l'extrusion peut être effectuée sont décrites dans les brevets EP 0667146, EP 0706790, EP 0745376 et EP 0755668.

Les exemples ci-après de compositions selon l'invention sont donnés à titre d'illustration. Les quantités y sont données en % en poids, sauf mention contraire.

### Exemple 1

| Préparation P | |
|---|---|
| Cire microcristalline | 13 % |
| Poudre de nylon | 6,52 % |
| Acide ascorbique | 10,86 % |
| Isononanoate d'isononyle | 13,04 % |
| Expancel | 0,22 % |
| Silice | 1,96 % |
| Polymethylsesquioxane | 2,17 % |
| Dimethicone/vinyl dimethicone crosspolymer and dimethicone (KSG 16 de la société Shin-Etsu) | 10,87 % |
| Isoparaffine hydrogénée | 41,36 % |

| Emulsion E/H | |
|---|---|
| Préparation P | 46 % |
| Huile de silicone | 4 % |
| Glycérine | 2 % |
| Polydiméthylsiloxane oxyéthyléné (DC2-5225C) | 8 % |
| Eau | qsp 100 % |

Mode opératoire : On prépare d'abord la préparation P dans un mélangeur-extrudeur et on obtient à la sortie du mélangeur-extrudeur, la préparation sous forme d'une pâte souple. On y incorpore le DC2-5225C et l'huile de silicone. On ajoute ensuite la phase aqueuse (glycérine et eau) à froid et sous agitation, dans le mélange obtenu. On obtient une émulsion souple.

## Revendications

1. Utilisation d'au moins 5 % en poids d'une ou plusieurs cires pour stabiliser un actif hydrophile choisi parmi la vitamine C et ses dérivés glycosylés et phosphatés dispersé dans la phase huileuse d'une composition comprenant plus de 5 % en poids d'eau, par rapport au poids total de la composition et une ou plusieurs charges.

2. Utilisation selon la revendication 1, **caractérisée en ce que** la cire présente une température de fusion commençante supérieure ou égale à 50°C, et mieux supérieure à 65°C.

3. Utilisation selon l'une des revendications 1 ou 2, **caractérisée en ce que** la quantité de cire(s) est d'au moins 5 % et va de préférence de 5 à 40 % et mieux de 5 à 20 % en poids par rapport au poids total de la composition.

4. Utilisation selon l'une quelconque des revendications précédentes, **caractérisée en ce que** l'actif hydrophile dispersé est la vitamine C.

5. Utilisation selon l'une quelconque des revendications précédentes, **caractérisée en ce que** la composition comprend au moins 7% en poids d'eau, avantageusement au moins 10 % d'eau par rapport au poids total de la composition.

6. Utilisation selon l'une quelconque des revendications précédentes, **caractérisée en ce que** la quantité de la phase huileuse, comprenant au moins 5 % en poids d'une ou plusieurs cires et l'actif hydrosoluble dispersé, va de 10 à 95 % en poids par rapport au poids total de la composition.

7. Utilisation selon l'une quelconque des revendications précédentes, **caractérisée en ce que** la composition est sous forme d'une émulsion.

8. Utilisation selon la revendication précédente, **caractérisée en ce que** la composition est sous forme d'une émulsion eau dans huile ou huile dans eau.

9. Utilisation selon l'une des revendications précédentes **caractérisée en ce que** la cire est choisie parmi les cires microcristallines, la paraffine, le pétrolatum, la vaseline, l'ozokérite, la cire de montan ; la cire d'abeilles, la lanoline ; les cires de Candellila, d'Ouricurry, de Carnauba, du Japon, le beurre de cacao, les cires de fibres de lièges ou de canne à sucre ; les cires de polyéthylène ; les cires de silicone, et leurs mélanges.

10. Utilisation selon l'une des revendications précédentes **caractérisée en ce que** la charge est choisie parmi le talc, les micas, le kaolin, les oxydes de zinc ou de titane, la carbonate de calcium, le carbonate et hydogénocarbonate de magnésium, la silice, les billes de verre et de céramique, les savons métalliques dérivés d'acide organique carboxylique ayant de 8 à 22 atomes de carbone, les poudres de polymères synthétiques non expansées, les poudres expancées, les poudres de matériaux organiques naturels et les microbilles de résine de silicone.

11. Utilisation selon l'une des revendications précédentes **caractérisée en ce que** la charge représente jusqu'à 20 %, de préférence de 1 à 12 % en poids par rapport au poids total de la composition.

## Claims

1. Use of at least 5% by weight of one or more waxes for stabilizing a hydrophilic active principle chosen from vitamin C and its glycosylate and phosphate derivatives dispersed in the oily phase of a composition comprising more than 5% by weight of water, with respect to the total weight of the composition, and one or more fillers.

2. Use according to Claim 1, **characterized in that** the wax exhibits a starting melting temperature of greater than or equal to 50°C and better still of greater than 65°C.

3. Use according to either of Claims 1 and 2, **characterized in that** the amount of wax(es) is at least 5% and preferably ranges from 5 to 40% and better still from 5 to 20% by weight with respect to the total weight of the composition.

4. Use according to any one of the preceding claims, **characterized in that** the dispersed hydrophilic active principle is vitamin C.

5. Use according to any one of the preceding claims, **characterized in that** the composition comprises at least 7% by weight of water, advantageously at least 10% of water, with respect to the total weight of the composition.

6. Use according to any one of the preceding claims, **characterized in that** the amount of the oily phase, comprising at least 5% by weight of one or more waxes and the dispersed water-soluble active principle, ranges from 10 to 95% by weight with respect to the total weight of the composition.

7. Use according to any one of the preceding claims, **characterized in that** the composition is in the form of an emulsion.

8. Use according to the preceding claim, **characterized in that** the composition is in the form of a water-in-oil or oil-in-water emulsion.

9. Use according to one of the preceding claims, **characterized in that** the wax is chosen from microcrystalline waxes, paraffin wax, petrolatum wax, ozokerite or montan wax; beeswax or lanolin; candelilla, ouricury, carnauba or Japan waxes, cocoa butter, or cork fibre or sugar cane waxes; polyethylene waxes; silicone waxes, and their mixtures.

10. Use according to one of the preceding claims, **characterized in that** the filler is chosen from talc, micas, kaolin, zinc or titanium oxides, calcium carbonate, magnesium carbonate and basic magnesium carbonate, silica, glass and ceramic beads, metal soaps derived from an organic carboxylic acid having from 8 to 22 carbon atoms, non-expanded powders formed of synthetic polymers, expanded powders, powders formed of natural organic materials, and silicone resin microbeads.

11. Use according to one of the preceding claims, **characterized in that** the filler represents up to 20%, preferably from 1 to 12%, by weight with respect to the total weight of the composition.

## Patentansprüche

1. Verwendung von mindestens 5 Gew.-% eines oder mehrerer Wachse zur Stabilisierung eines hydrophilen Wirkstoffs, der unter Vitamin C und seinen glykosylierten und phosphatierten Derivaten ausgewählt ist und in der Ölphase einer Zusammensetzung dispergiert ist, die bezogen auf das Gesamtgewicht der Zusammensetzung mehr als 5 Gew.-% Wasser und einen oder mehrere Füllstoffe enthält.

2. Verwendung nach Anspruch 1, **dadurch gekennzeichnet, dass** das Wachs eine Schmelzbeginn-Temperatur von größer oder gleich 50 °C und besser noch von über 65 °C aufweist.

3. Verwendung nach einem der Ansprüche 1 oder 2, **dadurch gekennzeichnet, dass** der Mengenanteil des Wachses (der Wachse) mindestens 5 Gew.-% beträgt und vorzugsweise im Bereich von 5 bis 40 Gew.-% und besser noch von 5 bis 20 Gew.-%, bezogen auf das Gesamtgewicht der Zusammensetzung, liegt.

4. Verwendung nach einem der vorhergehenden Ansprüche, **dadurch gekennzeichnet, dass** es sich bei dem dispergierten hydrophilen Wirkstoff um Vitamin C handelt.

5. Verwendung nach einem der vorhergehenden Ansprüche, **dadurch gekennzeichnet, dass** die Zusammensetzung mindestens 7 Gew.-% Wasser und vorteilhaft mindestens 10 Gew.-% Wasser, bezogen auf das Gesamtgewicht der Zusammensetzung, enthält.

6. Verwendung nach einem der vorhergehenden Ansprüche, **dadurch gekennzeichnet, dass** der Mengenanteil der Ölphase, die mindestens 5 Gew.-% eines oder mehrerer Wachse und den dispergierten wasserlöslichen Wirkstoff enthält, im Bereich von 10 bis 95 Gew.-%, bezogen auf das Gesamtgewicht der Zusammensetzung, liegt.

7. Verwendung nach einem der vorhergehenden Ansprüche, **dadurch gekennzeichnet, dass** die Zusammensetzung in Form einer Emulsion vorliegt.

8. Verwendung nach dem vorhergehenden Anspruch, **dadurch gekennzeichnet, dass** die Zusammensetzung in Form einer Wasser-in-Öl-Emulsion oder Öl-in-Wasser-Emulsion vorliegt.

9. Verwendung nach einem der vorhergehenden Ansprüche, **dadurch gekennzeichnet, dass** das Wachs unter mikrokristallinen Wachsen, Paraffin, Petrolatum, Vaseline, Ozokerit, Montanwachs, Bienenwachs, Lanolin, Candelillawachs, Ouricurywachs, Carnaubawachs, Japanwachs, Kakaobutter, Korkfaserwachs oder Zuckerrohrwachs, Polyethylenwachsen, Siliconwachsen und den Gemischen dieser Verbindungen ausgewählt ist.

10. Verwendung nach einem der vorhergehenden Ansprüche, **dadurch gekennzeichnet, dass** der Füllstoff unter Talk, Glimmern, Kaolin, den Oxiden von Zink oder Titan, Calciumcarbonat, Magnesiumcarbonat, Magnesiumhydrogencarbonat, Kieselsäure, Glaskugeln und Keramikkugeln, Metallseifen, die von einer organischen Carbonsäure mit 8 bis 22 Kohlenstoffatomen abgeleitet sind, nicht expandierten synthetischen Polymerpulvern, expandierten Pulvern, Pulvern von natürlichen organischen Stoffen und Mikrokugeln aus Siliconharz ausgewählt ist.

11. Verwendung nach einem der vorhergehenden Ansprüche, **dadurch gekennzeichnet, dass** der Füllstoff bis zu 20 Gew.-% und vorzugsweise 1 bis 12 Gew.-%, bezogen auf das Gesamtgewicht der Zusammensetzung, ausmacht.
